(19) Europäisches Patentamt
European Patent Office
Office européen des brevets

(11) EP 1 734 031 A1

(12) **EUROPEAN PATENT APPLICATION**
published in accordance with Art. 158(3) EPC

(43) Date of publication:
**20.12.2006 Bulletin 2006/51**

(21) Application number: **04738206.4**

(22) Date of filing: **04.06.2004**

(51) Int Cl.:
***C07C 65/32*** *(2006.01)* ***C07C 51/00*** *(2006.01)*
***A61K 31/19*** *(2006.01)* ***A61P 9/10*** *(2006.01)*

(86) International application number:
**PCT/CN2004/000602**

(87) International publication number:
**WO 2005/087701 (22.09.2005 Gazette 2005/38)**

(84) Designated Contracting States:
**AT BE BG CH CY CZ DE DK EE ES FI FR GB GR HU IE IT LI LU MC NL PL PT RO SE SI SK TR**

(30) Priority: **12.03.2004 CN 200410007520**

(71) Applicant: **TEAM ACADEMY OF PHARMACEUTICAL SCIENCE**
**Feng Tai District,**
**Beijing 100039 (CN)**

(72) Inventors:
- **LIU, Quanzhi**
**Feng Tai District,**
**Beijing 100039 (CN)**
- **YANG, Wenbin**
**Feng Tai District,**
**Beijing 100039 (CN)**
- **QIN, Hua**
**Feng Tai District,**
**Beijing 100039 (CN)**
- **ZHAO, Xingkai**
**Feng Tai District,**
**Beijing 100039 (CN)**
- **MA, Xisheng**
**Feng Tai District,**
**Beijing 100039 (CN)**

(74) Representative: **Schreiber, Christoph**
**Patentanwälte von Kreisler Selting Werner,**
**Postfach 10 22 41**
**50462 Köln (DE)**

(54) **NOVEL 2-(ALPHA-N-PENTANONYL)BENZOETES, THEIR PREPARATION AND USE**

(57) The present invention relates to novel compounds 2-(α-n-pentanonyl)benzoates, their preparation method, the pharmaceutical composition containing the same, and their use in preparing the medicament for preventing and treating cardio-cerebral ischemic disease, inhibiting thrombosis and alleviating the disturbance of cardio-cerebral circulation.

EP 1 734 031 A1

**Description**

FIELD OF THE INVENTION

The present invention relates to novel 2-(α-n-pentanonyl)benzoates, preparation method thereof, and pharmaceutical composition containing the same as active ingredient, and also relates to their use for preventing or treating cardio-cerebral ischemic diseases, alleviating the disturbance of cardio-cerebral circulation and inhibiting thrombosis, etc.

BACKGROUND OF THE INVENTION

Acute ischemic cerebral apoplexy and coronary heart disease, as well as myocardial infarction, are all diseases caused by ischemic injury induced by forming thrombosis due to various factors. The Patients suffering from these diseases are severely afflicted, and even to death. Currently, the medicaments for treating such diseases are being the focus of new medicament development.

Most of 3-alkylphthalides have pharmaceutical activities, one of which is known as DL-3-n-butylphthalide. Chinese Patent No. ZL9a117148.2 discloses the activity of DL-3-n-butylphthalide for the treatment of cerebral ischemia. DL-3-n-butylphthalide has come into the market in China as a Class One new drug in the trade name of "Enbipu", the dosage form of which is soft capsules. It is reported that 3-n-butenylphthalide has the effect of inhibiting the platelet aggregation (Biochimica et, Biophysica Acta, 924(1987), 375-382), but the activity of inhibiting cerebral ischemia and thrombosis has not been reported till now. Moreover, 3-n-butenylphthalide is also a viscous liquid which is insoluble in water as same as DL-3-n-butylphthalide, and thus it is not adapted to be formulated into a suitable formulation.

Chinese Application No. 01109795.7, for the first time discloses the preparation of 2-(α-hydroxypentyl)benzoates by using DL-3-n-butylphthalide as a precursor and the use thereof. The disclosed 2-(α-hydroxypentyl)benzoates involves the salt of a monovalent metal ion, a bivalent metal ion and an organic base, particularly the salt of K, Na, Ca, Mg, Zn, aniline, benzyl amine, morpholine and diethylamine. The description of the application discloses the preparation of potassium salt, sodium salt, lithium salt, calcium salt and benzyl amine salt, but does not disclose the preparation of zinc salt, aniline salt, morpholine salt and diethylamine salt. The description also discloses the effect of potassium salt on cerebral infarct area in rats having focal cerebral ischemia, the effect of potassium salt on the platelet aggregation in rats, and potassium salt preventing rats from cardioischemia-reperfusion induced arrhythmia, demonstrating the beneficial effect of potassium salt in the above-mentioned experiments. No pharmaceutical experiment is conducted with other salts to demonstrate their efficacy.

During the development of new drugs for treating cardio-cerebral vascular diseases, the inventors studied a large amount of 2-alkyl benzoates, and found that 3-n-butenylphthalide had the activity of inhibiting cerebral ischemia and thrombosis, and also unexpectedly found that 2-(α-n-pentanonyl)benzoates had more excellent activity on cardio-cerebral vascular and have more excellent physical and chemical performance as compared with 2-(α-hydroxypentyl) benzoates and the precursor 3-n-butenylphthalide known in the art.

SUMMARY OF THE INVENTION

Accordingly, an object of the present invention is to provide 2-(α-n-pentanonyl)benzoates, which were shown to significantly inhibit cerebral ischemia and thrombosis, and thereby improve cardio-cerebral circulation, prevent and treat cardio-cerebral ischemic diseases. The compounds have good appearance and physical form, and excellent stability to the light, heat and humidity.

Another object of the present invention is to provide a method for preparing 2-(α-n-pentanonyl)benzoates.

A further object of the present invention is to provide a pharmaceutical composition for clinically preventing or treating cardio-cerebral ischemic diseases, alleviating the disturbance of cardio-cerebral circulation and inhibiting thrombosis.

A still further object of the present invention is to provide the use of the above-mentioned compounds and composition in preparing the medicament for preventing and treating cardio-cerebral ischemic diseases, alleviating the disturbance of cardio-cerebral circulation and inhibiting thrombosis.

In order to achieve the objects of the present invention, the inventors have prepared and studied a large amount of 2-alkyl benzoates, including the salts disclosed in Chinese Application No. 01109795.7. After studying on their physical and chemical properties, pharmacodynamics and toxicity, the inventors unexpectedly found that the compounds having the following formula have excellent performance, which have not been reported in the prior art yet,

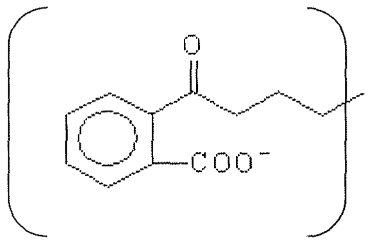

n $\cdot M^{n+}$

wherein n is 1 or 2; M is a monovalent metal ion such as $Li^+$, $Na^+$ and $K^+$, or a bivalent metal ion such as $Mg^{2+}$, $Ca^{2+}$ and $Zn^{2+}$, or an organic base group such as benzyl amine, t-butyl amine, methyl benzyl amine and N,N'-dibenzylethylenediamine. $Na^+$ and $K^+$ are preferred monovalent metal ions, $Ca^{2+}$ is a preferred bivalent metal ion, and N,N'-dibenzylethylenediamine is a preferred organic base group.

The compounds of the present invention may be prepared as following:

The preparing method in which M is an organic base group comprises the steps of: hydrolyzing 3-n-butenylphthalide (prepared in accordance with the method as described in the art, for example in Journal of Lanzhou University (Natural Science), 1990, 26(1): 118-119, *The synthesis of (±)-butylphthalide,* Li Shaobai and Zhang Nongming) under an alkaline condition; acidifying the hydrolyzed product to obtain 2-(α-n-pentanonyl)benzoic acid; dissolving the 2-(α-n-pentanonyl)benzoic acid in a solvent with low polarity and then reacting with an organic base, stirring for several hours, salting out, filtering and drying to obtain the 2-(α-n-pentanonyl)benzoates of the organic base in the form of white solid. The solvent with low polarity comprises benzenes (such as toluene), ethyl ether, dichloromethane, and ethyl acetate, preferably ethyl ether.

The preparing method in which M is a monovalent metal ion comprises the steps of: dissolving 2-(α-n-pentanonyl)benzoic acid in a polar solvent and then reacting with a metal ionic base to form a salt, adding a solvent with low polarity under stirring, stirring continuously for several hours, salting out, filtering and drying to obtain 2-(α-n-pentanonyl)benzoates of monovalent metal ion in the form of white solid. The polar solvent comprises C1-C4 lower alcohols, preferably methanol. The solvent with low polarity comprises benzenes (such as toluene), ethyl ether, dichloromethane, and ethyl acetate, preferably ethyl ether.

The preparing method in which M is a bivalent metal ion comprises the steps of: mixing the prepared 2-(α-n-pentanonyl)benzoates with the solution of a bivalent metal ionic salt, performing trans-salification reaction to obtain 2-(α-n-pentanonyl)benzoates of bivalent metal ion. The suitable solvent comprises commonly used polar solvent, such as water and alcohols, preferably water and C1-C4 lower alcohols such as methanol and ethanol.

The inventors have found that the compounds of the present invention, particularly the preferred compounds, have the following excellent performance:

- good physical form, which is adapted to be formulated into a formulation;
- only simple post-treatment is needed to achieve a pharmaceutically accepted purity;
- the compounds of the present invention are more stable in comparison with the already disclosed 2-(α-hydroxypentyl) benzoates as demonstrated by the stability test;
- the compounds of the present invention are less toxic than the already disclosed 2-(α-hydroxypentyl)benzoates as demonstrated by the acute toxicity test in animal;
- in pharmacodynamics test *in vivo,* as compared with 2-(α-hydroxypentyl)benzoates and 3-n-butenylphthalide known in the art, the compounds of the present invention at the same dosage have more excellent protection effect on the injury of brain tissue induced by cerebral artery occlusion, and may significantly alleviate the neural symptoms, significantly decrease the infarct area in model rats of middle cerebral artery occlusion, and significantly inhibit the formation of thrombosis of artery-vein bypass, demonstrating that the compounds of the present invention may effectively inhibit thrombosis, and have relatively good preventive and therapeutic effect on cardio-cerebral ischemic disease;
- in the experiment for testing the effect on neural symptoms and cerebral infarct area in model rats of middle cerebral artery occlusion, as compared with 2-(α-hydroxypentyl)benzoates and 3-n-butenylphthalide known in the art, the compounds of the present invention has lower dosage need for significant therapeutic efficacy. That is to say, the

compounds of the present invention may have significant efficacy at a relatively lower dosage.

The pharmaceutical composition of the present invention comprises a therapeutically effective amount of the compounds according to the present invention as active ingredients, and one or more pharmaceutically acceptable carriers.

The pharmaceutically acceptable carriers as described above, refer to the conventional pharmaceutical carriers used in the pharmaceutical art (also known as adjuvant), wherein the adjuvant generally used in the solid formulation includes fillers such as starch, sucrose, and the like; adhesives such as cellulose derivatives, alginates, gelatin, and polyvinylpyrrolidone; wetting agents such as ethanol and water; disintegrants such as starch and the derivatives thereof, such as sodium carboxymethyl starch (CMS-Na), low substituted hydroxypropyl cellulose (LS-HPC), crosslinked polyvinylpyrrolidone, crosslinked sodium carboxymethyl cellulose (CCMC-Na), and the like and effervescency disintegrants; absorbance promoting agents such as quaternary ammonium compounds; surfactants such as hexadecanol; adsorbant carriers such as kaolin and bentonite; lubricants such as talc, calcium and magnesium stearate, polyethylene glycol. In addition, other adjuvant such as flavoring agents, sweeting agents and the like may also be added into the composition. The adjuvant used in the liquid formulation includes water for injection, and pH adjusting agents such as sodium hydroxide, as well as other adjuvant such as isotonic adjusting agents. Those skilled in the art may rationally select the type and amount of the generally used adjuvant in accordance with conventional methods for preparing liquid formulations known in the pharmaceutical art, so as to allow the final product meet the requirements for conventional intravenous injections or lyophilized intravenous injections.

The compounds and the pharmaceutical composition according to the present invention may be used to manufacture medicaments for preventing and treating cardio-cerebral ischemic diseases, inhibiting thrombosis, and alleviating the disturbance of cardio-cerebral circulation.

Various dosage forms of the pharmaceutical composition according to the present invention may be prepared by those skilled in the art by using the conventional preparation methods known in the pharmaceutical art. For example, those skilled in the art may mix the active ingredient with one or more carriers, and then formulate into the desired dosages forms, including tablets, capsules and granules. Furthermore, those skilled in the art may also formulate water-soluble salts of the compounds according to the present invention into intravenous injections and lyophilized intravenous injections in accordance with the conventional methods for preparing intravenous injections known in the pharmaceutical art.

## DETAILED DESCRIPTION OF THE INVENTION

The following examples may be helpful for those skilled in the art to fully understand the present invention, but should not be construed to limit the scope of the present invention in any way.

Example 1: The preparation of 2-(α-n-pentanonyl)benzoic acid

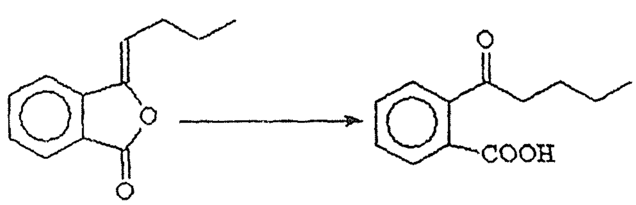

100 g (0.53 mol) of 3-n-butenylphthalide, 300 ml of methanol, 40 g of NaOH and 50 ml of water were added into a flask, refluxed for 8 hours. Methanol was removed under reduced pressure and 200 ml of water was supplemented, and then cooled. Hydrochloride solution at a concentration of 4 N was added in dropwise with stirring, so as to adjust pH in the range of 2 to 3. The reaction mixture was extracted with ethyl ether. Next, the extracted solution was washed with water, dried and filtered. Then ethyl ether was removed under reduced pressure, to obtain 100 g of yellowish colloidal solid, with a yield of 91 %.

Example 2: The preparation of sodium 2-(α-n-pentanonyl)benzoate

(1)

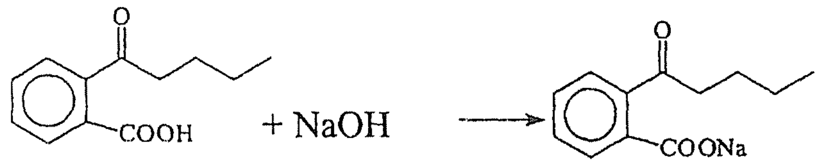


3.4 g (0.085 mol) of NaOH, 50 ml of methanol, and 21 g (0.1 mol) of 2-(α-n-pentanonyl)benzoic acid were added into a flask, stirred at the room temperature for 2 hours. 1000 ml of ethyl ether was added in batches, so as to allow a large amount of white solid appears. Filtering and drying were performed after stirring for 2 hours. The obtained solid was washed with ethyl ether, and filtering was conducted. The filter cake was then vacuum dried to obtain 16 g of sodium 2-(α-n-pentanonyl)benzoate as a white powdered crystal, with a yield of 82.6% (as calculated by NaOH), m. p. : 219.0-220.0˚C.

(2) The procedure was the same as that in step (1) except that methanol was replaced with equivalent volume of ethanol. 15 g of white powdered solid was obtained, with a yield of 77.4%, m. p. :219.0-220.5˚C.

(3) The procedure was the same as that in step (1) except that ethyl ether was replaced with equivalent volume of toluene, dichloromethane, and ethyl acetate respectively. The yields were all somewhat decreased.

Infrared absorption spectrum: KBr, 3060cm$^{-1}$, ($\gamma_{Ar-H}$). 1689 cm$^{-1}$, ($\gamma_{C=O}$),
$^{1}$H NMR: (300MHz,$CD_3Cl$) δ(ppm)6.999-7.806(m, 4H, Ar-H), 2.485-2.689(1, 2H, $-CH_2-$), 1.455-1.530(m, 2H, $-CH_2-$), 1.223-1.295(m, 2H, $-CH_2-$) 0.805-0.88.50 (t, 3H, $-CH_3$)

ESI-MS: Negative M/Z 205

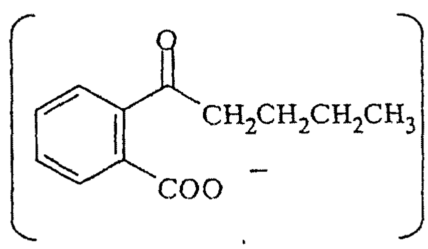

Element analysis: $C_{12}H_{13}NaO_3$ FW228.22

| | C | H |
|---|---|---|
| % theoretical value | 63.15 | 5.74 |
| % measured value | 63.20 | 5.80 |

Example 3: The preparation of potassium 2-(α-n-pentanonyl)benzoate

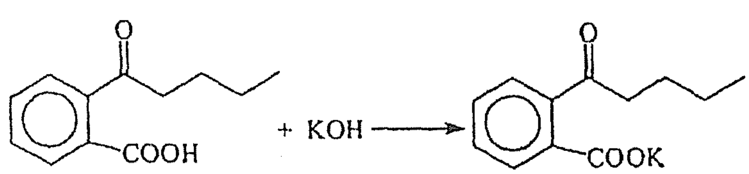

4.5 g (0.08 mol) of KOH, 50 ml of methanol, and 21 g (0.10 mol) of 2-(α-n-pentanonyl)benzoic acid were added into a flask, stirred at the room temperature for 2 hours. The solvent was removed under reduced pressure. 1.0 liter of anhydrous ethyl ether was added and stirred so as to allow a large amount of white solid appears. Filtering was performed after stirring for 2 hours. The solid was washed with ethyl ether, followed by removing ethyl ether under reduced pressure. Next, vacuum drying was conducted to obtain 16 g of white solid, with a yield of 81.8% (as calculated by KOH), m. p. : 113.5-115.0°C.

Infrared absorption spectrum: KBr, 3062 $cm^{-1}$, ($\gamma_{Ar-H}$).1685 $cm^{-1}$, ($\gamma_{C=O}$)
$^1H$ NMR: (400MHz,$CD_3Cl$) δ(ppm) 6.9550-7.7305(m,4H,Ar-H), 2.6099-2.6476(t, 2H, $-CH_2-$), 1.4531-1.5288(m, 2H, $-CH_2-$), 1.2116-1.23043(m, 2H, $-CH_2-$)0.8141-0.8508 (t, 3H, $-CH_3$)

ESI-MS: Negative M/Z 205

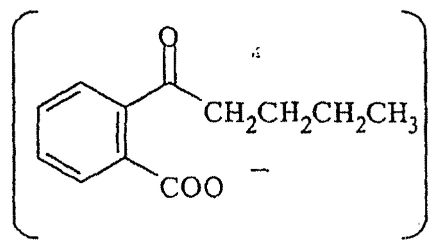

Element analysis: $C_{12}H_{13}KO_3$ FW244.33

| | C | H |
|---|---|---|
| % theoretical value | 58.99 | 5.36 |
| % measured value | 58.90 | 5.40 |

Example 4: The preparation of calcium 2-(α-n-pentanonyl)benzoate

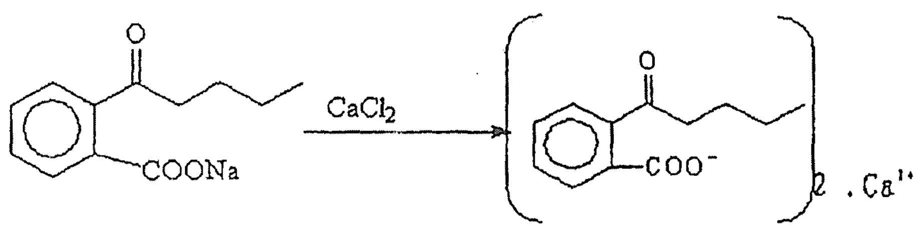


44 g (0.193mol) of sodium 2-(α-n-pentanonyl)benzoate and 95 ml of water were added into a beaker, stirred at the room temperature to allow thorough dissolution. Then 10.7 g (0.096 mol) of $CaCl_2$ was dissolved in 25 ml of water, and the resulting solution was added in batches into the stirred aqueous solution of said sodium salt, so as to allow a white solid appears immediately. Filtering was performed after stirring for 2 hours at room temperature. The filter cake was washed with 60 ml of water, and then with ethyl ether. Then drying was conducted to obtain 37 g of white solid, with a yield of 85.5%, m. p.:108.0-110.5°C.

Infrared absorption spectrum: KBr,3062$cm^{-1}$, (γ Ar-H).1687 $cm^{-1}$, (γ C=O)
$^1H$ NMR: (400MHz, CD3Cl) δ(ppm) 7.0392 - 7.8998 (m, 8H, Ar-H), 2.6507 - 2.6885 (t, 4H, -CH2-), 1.4591 - 1.5346 (m, 4H, -CH2-), 1.1921 - 1.2846 (m, 4H, -CH2-), 0.7960 - 0.8325 (t, 6H, -CH3)

ESI-MS: Negative M/Z 205

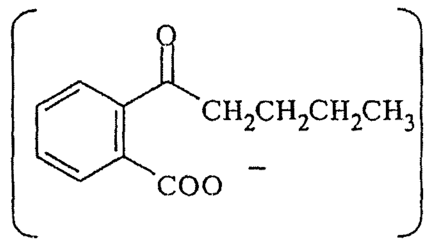

Element analysis: $C_{24}H_{26}CaO_6$ FW450.54

| | C | H |
|---|---|---|
| % theoretical value | 63.98 | 5.82 |
| % measured value | 63.90 | 5.80 |

Example 5: The preparation of 2-(α-n-pentanonyl)benzoate of N,N'-dibenzylethylene diamine

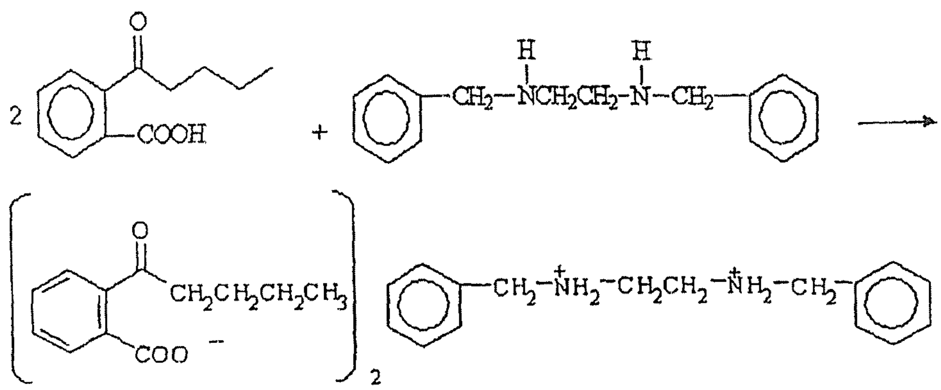

21.4 g (0.089 mol) of N,N'-dibenzylethylenediamine and 1000 ml of ethyl ether were added into a flask, stirred at the room temperature to allow thorough dissolution. Then a solution of 36.6g (0.178 mol) of 2-(α-n-pentanonyl)benzoic acid in 1000 ml of ethyl ether was added into the above obtained solution, to allow the solution turn cloudy, and then to allow a large amount of white solid appear after the addition was accomplished. Filtering was performed after stirring at the room temperature for 2 hours. The filter cake was washed with ethyl ether. Then drying was conducted to obtain 39 g of white solid, with a yield of 66.9%, m. p. :94.5-96.5 °C .

Infrared absorption spectrum: KBr, 3030, 3060 $cm^{-1}$, (γ Ar-H).1685 cm-1, (γ C=O)

$^1H$ NMR: (400MHz, $CD_3Cl$) δ (ppm) 7.2.526 - 7.7949 (m, 18H), 3.8.531 (S, 4H, two Ar-$CH_2$), 2.8133 (s, 4H, N-$CH_2CH_2$-N), 2.4140 - 2.49811(m, 4H, two -$CH_2$-), 1.3312 - 1.4052 (m, 4H, two -$CH_2$-), 1.0866 - 1.3004 (m, 4H, two -$CH_2$-), 0.7994 - 0.8356 (m, 6H, two -$CH_3$)

ESI-MS: Negative M/Z 205

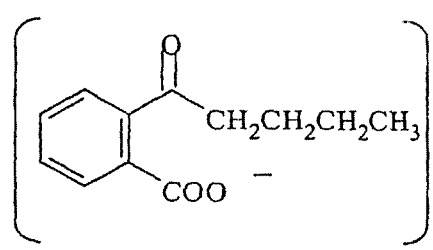

Positive M/Z 241

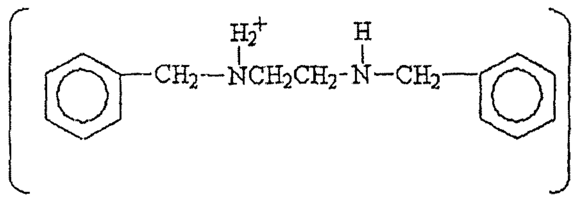

Element analysis: $C_{40}H_{50}N_2O_6$ FW654.85

| | C | H | N |
|---|---|---|---|
| % theoretical value | 73.37 | 7.70 | 4.28 |
| % measured value | 73.20 | 7.85 | 4.30 |

Example 6: The preparation of 2-(α-n-pentanonyl)benzoate of t-butyl amine

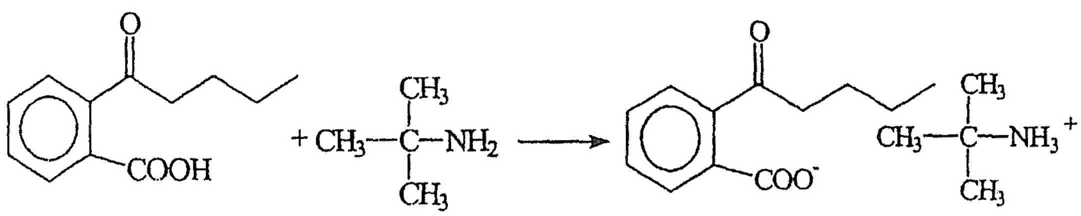

17.8 g (0.243 mol) of t-butyl amine was dissolved in 1000 ml of ethyl ether, then a solution of 50 g (0.243 mol) of 2-(α-n-pentanonyl)benzoic acid in 1000 ml of ethyl ether was added. The reaction was stirred at the room temperature for 15 minutes to allow a large amount of white solid appears. Stirring was continued for 2 hours, followed by filtering. The filter cake was washed with ethyl ether twice. Then drying was conducted to obtain 50 g of white solid, with a yield of 73.5%, m. p.:122.5-124.0˚C.

Infrared absorption spectrum: KBr, 3060 $cm^{-1}$, ($\gamma_{Ar-H}$).1683 $cm^{-1}$, ($\gamma_{C=O}$)

ESI-MS: Negative M/Z 205

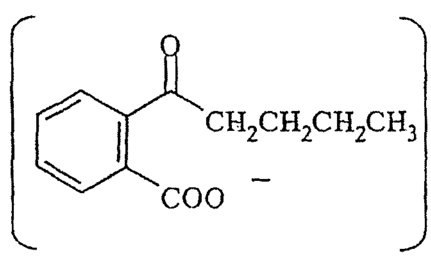

Element analysis: $C_{16}H_{25}NO_3$ FW279.38

| | C | H | N |
|---|---|---|---|
| % theoretical value | 68.79 | 9.02 | 5.01 |
| % measured value | 68.83 | 9.05 | 4.97 |

Example 7: The preparation of 2-(α-n-pentanonyl)benzoate of benzyl amine

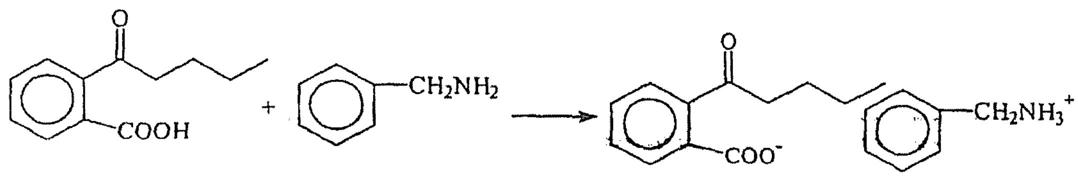

25 g (0.121 mol) of 2-(α-n-pentanonyl)benzoic acid was dissolved in 700 ml of ethyl ether, and then 12.8 g (0.120 mol) of benzyl amine was added. The reaction was stirred at the room temperature for 10 minutes to allow white solid appears. Stirring was continued for 2 hours, followed by filtering. The filter cake was washed with ethyl ether and dried to obtain 35 g of white solid, with a yield of 93%, m. p. :72.5-74.0˚C.

ESI-MS: Negative M/Z 205

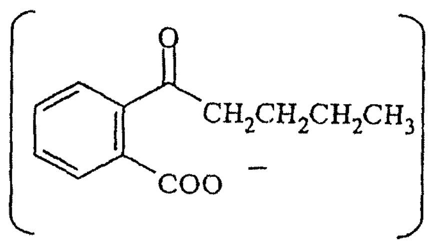

Element analysis: $C_{19}H_{23}NO_3$ FW313.40

| | C | H | N |
|---|---|---|---|
| % theoretical value | 72.82 | 7.40 | 4.47 |
| % measured value | 72.90 | 7.35 | 4.51 |

Example 8: The preparation of 2-(α-n-pentanonyl)benzoate of (S)-α-methyl benzyl amine

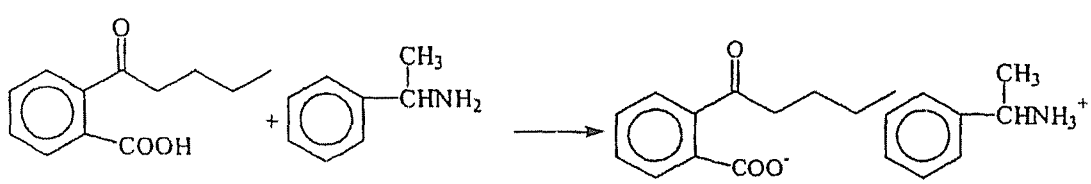

2.5 g (0.121 mol) of 2-(α-n-pentanonyl)benzoic acid was dissolved in 700 ml of ethyl ether in a flask, and then 14.6 g (0.120 mol) of (S)-α-methyl benzyl amine was added. The reaction was stirred at the room temperature for 10 minutes to allow white solid appears. Stirring was continued for 2 hours, followed by filtering. The filter cake was washed with ethyl ether and dried to obtain 30 g of white solid, with a yield of 76.4%, m. p. :82.0-84.0 ˚C.

ESI-MS: Negative M/Z 205

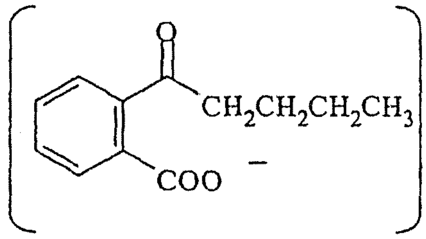

Element analysis: $C_{20}H_{25}NO_3$ FW327.41

| | C | H | N |
|---|---|---|---|
| % theoretical value | 73.14 | 7.98 | 4.26 |
| % measured value | 73.10 | 8.05 | 4.28 |

Example 9: The preparation of zinc 2-(α-n-pentanonyl)benzoate

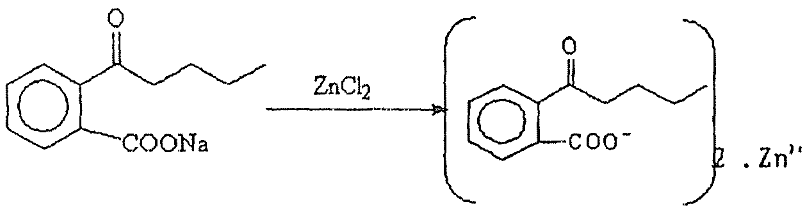


44 g (0.19.3 mol) of sodium 2-(α-n-pentanonyl)benzoate were added into 95 ml of water in a beaker to be completely dissolved under stirring. 13.2 g (0.096 mol) of $ZnCl_2$ was dissolved in 125 ml of water and the resulting solution was cooled to the room temperature. The resulting solution was added in batches into the stirred aqueous solution of the above sodium salt, so as to allow white viscous colloid appears immediately. Filtering was conducted after stirring at the room temperature for 2 hours to obtain the white viscous colloid, which then was vacuum dried to obtain white colloidal solid.

Example 10: The preparation of magnesium 2-(α-n-pentanonyl)benzoate

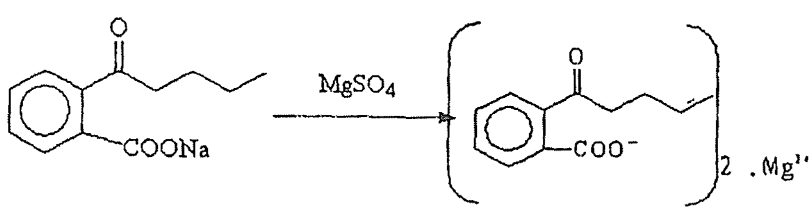


44 g (0.193 mol) of sodium 2-(α-n-pentanonyl)benzoate were added into 95 ml of water in a beaker to be completely dissolved under stirring at room temperature. 11.5 g (0.096 mol) of $MgSO_4$ was dissolved in 125 ml of water and the resulting solution was cooled to the room temperature, and then added in batches into the stirred aqueous solution of the above sodium salt, so as to allow white viscous colloid appears. Filtering was conducted after stirring at the room temperature for 2 hours to obtain the white viscous colloid, which then was vacuum dried to obtain white foam solid.

Example 11: The preparation of lithium 2-(α-n-pentanonyl)benzoate

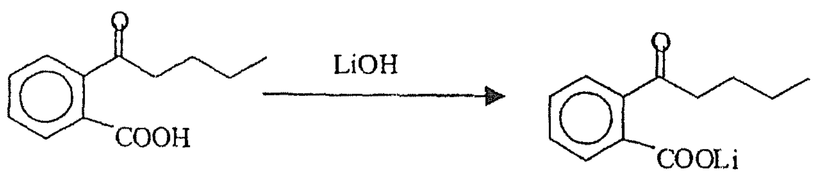


2.0 g (0.085 mol) of LiOH, 50 ml of methanol, and 21 g (0.1 mol) of 2-(α-n-pentanonyl)benzoic acid were added into a flask, stirred at the room temperature for 2 hours. 1000 ml of ethyl ether was added in batches, so as to allow a large amount of white viscous material appears. Filtering and drying were performed after stirring for 2 hours. The viscous material was washed with ethyl ether, and filtering was conducted. The filter cake was then vacuum dried to obtain white foam solid.

Summary: lithium salt, magnesium salt and zinc salt are all foam or colloidal solid without a specific melting point, and thus do not meet the requirement of pharmaceutical industry. Sodium salt, potassium salt, calcium salt, salt of benzyl amine, salt of N,N'-dibenzylethylenediamine and salt of t-butyl amine all have good physical appearance and specific melting point.

Example 12. The stability of aqueous solution

Compounds were dissolved in water to form aqueous solutions in a concentration of 0.2 mg/ml, and the amount of decomposed products were measured. The results were shown in Table 1.

Table 1. The comparison of stability of aqueous solution

| Time / wt % decomposed product / Sample | 0 hr | 1 hr | 2 hr | 4 hr | 16 hr | 24 hr |
|---|---|---|---|---|---|---|
| Sodium 2-(α-n-pentanonyl)benzoate | 0 | 0 | 0 | 0 | 0 | 0 |
| Potassium 2-(α-n-pentanonyl)benzoate | 0 | 0 | 0 | 0 | 0 | 0 |
| Potassium 2-(α-hydroxypentyl)benzoate * | 0 | 0.2 | 0.36 | 0.75 | 4.5 | 5.3 |

*: 2-(α-hydroxypentyl)benzoates used in the Example were all prepared by using the methods described in CN1382682A, similarly hereinafter.

The amounts of decomposed products were determined in accordance with the test described in Pharmacopoeia of P.R. China, 2000 edition, appendix VD of part two, by HPLC method, C18 column, mobile phase of acetonitrile:0.02M sodium dihydrogen phosphate = 40:60, detecting wavelength of 230 nm, flow rate of 1 ml/min.

The results showed that no decomposed product of sodium 2-(α-n-pentanonyl)benzoate and potassium 2-(α-n-pentanonyl)benzoate may be detected after exposed to water for 24 hours. The stability of these salts in aqueous

solution was significantly better than that of potassium 2-(α-hydroxypentyl)benzoate.

Example 13. The stability to humidity

Sodium 2-(α-n-pentanonyl)benzoate, potassium 2-(α-n-pentanonyl)benzoate, calcium 2-(α-n-pentanonyl)benzoate and 2-(α-n-pentanonyl)benzoate of N,N'-dibenzyl ethylenediamine, together with potassium 2-(α-hydroxypentyl) benzoate, calcium 2-(α-hydroxypentyl)benzoate and 2-(α-hydroxypentyl)benzoate of N,N'-dibenzyl ethylenediamine were all tested for 10 days in accordance with the testing method as described in Pharmacopoeia of P.R. China, 2000 edition, appendix XIX C of part two, "Guideline for testing the stability of medicament: method for high humidity test". The purity was determined in accordance with the test described in Pharmacopoeia of P.R. China, 2000 edition, appendix VD of part two, by using HPLC method, C18 column, mobile phase of acetonitrile:0.02M sodium dihydrogen phosphate = 40:60, detecting wavelength of 230 nm, flow rate of 1 ml/min.

The results showed that 2-(α-n-pentanonyl)benzoates slightly absorbed moisture in a condition of certain humidity, but these salts almost may not be decomposed after absorbing moisture, while potassium 2-(α-hydroxypentyl) benzoate was highly decomposed. N,N'-dibenzylethylenediamine salts of both acids were shown to absorb less moisture and were stable.

Example 14. The stability to light

Tests were conducted for 10 days in accordance with the testing method as described in Pharmacopoeia of P.R. China, 2000 ed., appendix XIX C of part two, "Guideline for testing the stability of medicament: method for testing stability to light". The purity was determined in accordance with the test described in Pharmacopoeia of P.R. China, 2000 ed., appendix VD of part two, by using HPLC method, C18 column, mobile phase of acetonitrile:0.02M sodium dihydrogen phosphate = 40:60, detecting wavelength of 230 nm, flow rate of 1 ml/min. The results showed that no decomposed product of sodium, potassium, calcium and N,N'-dibenzylethylenediamine 2-(α-n-pentanonyl)benzoates may be detected, indicating that their stability to light is good.

Example 15. Thermal stability

Tests were conducted for 10 days at 60°C in accordance with the testing method as described in Pharmacopoeia of P.R. China, 2000 ed., appendix XIX C of part two, "Guideline for testing the stability of medicament: method for testing thermal stability". The purity was determined in accordance with the test described in Pharmacopoeia of P.R. China, 2000 ed., appendix VD of part two, by using HPLC method, C18 column, mobile phase of acetonitrile:0.02M sodium dihydrogen phosphate = 40:60, detecting wavelength of 230 nm, flow rate of 1 ml/min. The results showed that no decomposed product of sodium, potassium, calcium and N,N'-dibenzylethylenediamine 2-(α-n-pentanonyl)benzoates may be detected, indicating that their thermal stability is good.

Example 16: Tablets

| Compositions | Dosage (mg per tablet) |
|---|---|
| 2-(α-n-pentanonyl)benzoates | 100 |
| Starch | 50 |
| Microcrystal cellulose | 35 |
| Magnesium stearate | 2 |
| Sodium carboxymethyl cellulose | 5 |

Preparing method: the active ingredients, starch, microcrystal cellulose and sodium carboxymethyl cellulose were mixed in the above predetermined ratio, wetted with water, granulated, dried and sieved. Then magnesium stearate was added. After mixing, the mixture was compressed to obtain the tablets of the present invention.

Example 17: Capsules

| Compositions | Dosage (mg per capsule) |
|---|---|
| 2-(α-n-pentanonyl)benzoates | 100 |
| Starch | 50 |
| Sodium carboxymethyl cellulose | 3 |
| Crosslinked PVP | 1 |
| Magnesium stearate | 1.5 |

Preparing method: the active ingredients and adjuvant were mixed in the above predetermined ratio, wet granulated, dried and sieved. Then magnesium stearate was added. After mixing, the mixture was filled into gastric soluble hard capsules to obtain the capsules of the present invention.

Example 18: Granules

| Compositions | Dosage (mg per bag) |
|---|---|
| 2-(α-n-pentanonyl)benzoates | 100 |
| Starch | 1000 |
| Dextrin | 150 |
| Sugar powder | 150 |

Preparing method: the active ingredients and adjuvant were mixed in the above predetermined ratio, wet granulated, dried, sieved, graded and packaged in fractional dose to obtain the granules of the present invention.

Example 19: Intravenous injections

| Compositions | Dosage |
|---|---|
| 2-(α-n-pentanonyl)benzoates | 100 mg per vial |
| Sodium hydroxide | Appropriate amount |
| Water for injection | Appropriate amount |
| Sodium chloride | Appropriate amount |

Preparing method: water soluble 2-(α-n-pentanonyl)benzoates was dissolved in appropriate amount of water for injection, appropriate amount of sodium hydroxide was added to adjust pH to 9.0 (the range may be between 7.5 to 9.5). Appropriate amount of sodium chloride was added if desired. The resulting solution was filled into vials and sterilized to obtain the intravenous injections of the present invention.

Example 20: lyophilize intravenous injections

| Compositions | Dosage |
|---|---|
| 2-(α-n-pentanonyl)benzoates | 100 mg per vial |
| Sodium hydroxide | Appropriate amount |
| mannitol | Appropriate amount |

Preparing method: water soluble 2-(α-n-pentanonyl)benzoates was dissolved in appropriate amount of water for injection, appropriate amount of sodium hydroxide was added to adjust pH to 9.0 (the range may be between 7.5 to 9.5). Appropriate amount of mannitol was added if desired. The resulting solution was filtered, filled into vials and lyophilized to obtain the lyophilized intravenous injections of the present invention, which may be diluted with 0.9% normal saline or 5% glucose injection solution for intravenous injection or infusion.

Example 21: Acute toxicity test

This Example used the following:

1. Animals

ICR mice (one half of which are females and another half are males), whose body weights were 18-20g, were provided by Vital River, Beijing. The certificate of approval was SCXK 2002-0003.

2. Test drugs

3-n-butenylphthalide; sodium, potassium, calcium and N,N'-dibenzylethylenediamine 2-(α-n-pentanonyl)benzoates; DL-3-n-butylphthalide; potassium, calcium and N,N'-dibenzylethylenediamine 2-(α-hydroxypentyl)benzoates, were all provided by Teamacademy of Pharmaceutical Science, and were formulated into solutions of various concentrations with eatable salad oil.

3. Testing method

ICR mice were randomly divided into groups depending on body weight and gender, with 10 mice per group, 5 females and 5 males. In accordance with the results of experiments conducted in advance, the dosage of each group was designed with the interval between groups of 1:0.8. The test drugs were orally administered by gavage in a volume of 0.2 ml per 10 g body weight. Mice were provided merely with water 12 hours prior to administration, and normally fed after administration. Mice were observed for continuous 7 days, and toxic response and occurrence of death were recorded. Postmortem examination was conducted on the dead animals and pathological changes were macroscopically observed. $LD_{50}$ value and 95% confidence interval were calculated by the method of Bliss.

4. Test results

Table 2. Results of acute toxicity test

| Compounds | $LD_{50}$ value (g/kg) | 95% confidence interval (g/kg) |
|---|---|---|
| 3-n-butenylphthalide | 2.5692 | 2.3265-2.8377 |
| Sodium 2-(α-n-pentanonyl)benzoate | 2.6515 | 2.3957-2.9346 |
| Potassium 2-(α-n-pentanonyl)benzoate | 2.7248 | 2.4284-3.0573 |
| Calcium 2-(α-n-pentanonyl)benzoate | 2.6838 | 2.3716-3.0371 |
| N,N'-dibenzylethylenediamine 2-(α-n-pentanonyl)benzoate | 3.0788 | 2.7701-3.4220 |
| DL-3-n-butylphthalide | 2.1861 | 1.9798-2.4139 |
| Potassium 2-(α-hydroxypentyl)benzoate | 1.2272 | 1.1027-1.3659 |
| Calcium 2-(α-hydroxypentyl)benzoate | 1.5247 | 1.3405-1.7342 |
| N,N'-dibenzylethylenediamine 2-(α-hydroxypentyl)benzoate | 2.8089 | 2.5381-3.1085 |

It may be seen from the above-mentioned results that the acute toxicity of 3-n-butenylphthalide was lower than that of DL-3-n-butylphthalide, and the acute toxicity of potassium, calcium and N,N'-dibenzylethylenediamine 2-(α-n-pentanonyl)benzoates was lower than that of potassium, calcium and N,N'-dibenzylethylenediamine 2-(α-hydroxypentyl) benzoates, respectively.

Example 22. Effects on neural symptoms and cerebral infarct area in model rats of middle cerebral artery thrombosis (MCAT)

This example used the following:

1. Animals

Female and male SD rats, weighed 190-210g, were provided by Vital River, Beijing. The certificate of approval was SCXK 11-00-0008.

2. Drugs and reagents

Test drugs: 3-n-butenylphthalide; sodium, potassium, calcium, and N,N'-dibenzylethylenediaunine 2-($\alpha$-n-pentanonyl)benzoates; DL-3-n-butylphthalide; potassium, calcium and N,N'-dibenzylethylenediamine 2-($\alpha$-hydroxypentyl) benzoates, were all provided by Teamacademy of Pharmaceutical Science, and were formulated into solutions of various concentrations with eatable salad oil.

Reagents: $FeCl_3 \cdot 6H_2O$(A.R.), which was produced by Beijing Chemical Plant, was formulated with 1 mol/L of hydrochloride solution; Triphenyl tetrazolium Chloride (TCC) was produced by Beijing Chemical Plant.

3. Instruments

XTT microscope, produced by Beijing Dian-Guang Scientific Instrument Plant; model SHZ-22 thermostat water bath oscillator, produced by Jiangsu Tai-Cang Medical Instrument Plant; Electronic analytical balance, model AEG-220, produced by SHIMADZU, Japan.

4. Testing methods and results

A. Effects on neural symptoms of MCAT rats

(1) Grouping and administration: The experimental animals were divided into following groups, which were pseudo-operation group, MCAT model group, 50 mg/kg, 100 mg/kg and 200 mg/kg groups for each of the following: 3-n-butenylphthalide; sodium, potassium, calcium and N,N'-dibenzylethylenediamine 2-($\alpha$-n-pentanonyl)benzoates; DL-3-n-butylphthalide; potassium, calcium and N,N'-dibenzylethylenediamine 2-($\alpha$-hydroxypentyl)benzoates. Preventive administration was conducted by gavage one time per day for 3 days prior to the model being established. The model was established on day 4, and a dosage of one day was administered by gavage immediately after the model was established. Another dosage of one day was again administered by gavage 12 hours after the model was established. Equivalent amount of the vehicle was administered to the control group.

(2) Rats were anesthetized by intraperitoneally injecting 12% chloral hydrate (350 mg/kg) by following the modified method of Tamura et al (Tamura A, Graham DI, McCulluoch J et al. Focal Cerebral ischemia in the rat. 1. Description of technique and early neuropathological consequences following middle cerebral artery occlusion. J Cereb Blood Flow Metab, 1981, 1: 53), and fixed in lateral decubitus position. A bowtype incision of 1.5 cm long was made at the midpoint between canthus and exterior auditory canal. Temporalis was broken and incised to expose temporal bone. A window of 2.5 mm diameter was drilled by using a dentistry drill at a position of 1 mm under the anterior joint between jugal bone and the squamous part of temporal bone, so as to expose the middle cerebral artery (between olfactory tract and inferior cerebral vein). A little piece of filter paper that had absorbed 10 $\mu$l of 50% $FeCl_3$ solution was applied onto the middle artery. The filter paper was removed after 30 min when the vascular became black. The localized tissue was washed with physiological brine, and sutured layer by layer. Then rats were placed back into the cages.

3. Behavioral examination

By following the modified method of Bederson et al. (Bederson JB, Pitts LH, Tsuji M et al. Rat middle cerebral artery occlusion: evaluation of the mode and development of a neurologic examination. Stroke, 1986, 17: 472), the behavior of the animals was scored at different time point post the operation (6 h, 24 h). (1) The tail of the rat was lifted 30cm high away from the ground, and the flexion of the forelimbs was observed. "0" was recorded if the forelimbs

symmetrically stretch towards the ground; and "1" was recorded if shoulder flexion, elbow flexion and shoulder intorsion occured to the contralateral forelimbs, or both flextion and intorsion of the carpus and elbow occured. (2) The animals were placed onto a smooth ground, and the resistance of contralateral shoulder to push was examined. "0" was recorded if the resistance of both shoulders were equal and strong; and "1" was recorded if the resistance of contralateral shoulder to push was decreased. (3) The forelimbs of the animals were placed onto metal gauze and the tensile force of forelimbs muscle was observed. "0" was recorded if the tensile force on both sides were equal and strong; and "1" was recorded if the tensile force of contralateral forelimbs muscle was decreased. (4) The tail of the rat was lifted 30 cm high away from the ground, and "1" was recorded if the animals ceaselessly circled contralaterally. "4" was full mark in accordance with the above-mentioned standard. The higher the score was, the more severe the behavioral disorder of the animal was.

4. Results: The behavioral scores in each group were compared by student t test. The results were shown in tables 3, 4, and 5.

Table 3. Effects of compounds (50 mg/kg) on the neural symptoms of MCAT rats

| Groups | Dosage (mg/kg) | n | Scores | | % inhibition at hour 24 |
|---|---|---|---|---|---|
| | | | 6 hours | 24 hours | |
| Model group | - | 11 | 3.55±0.52 | 3.64±0.50 | - |
| Pseudo-operation group | - | 11 | 0.00±0.00*** | 0.00±0.00*** | - |
| 3-n-butenylphthalide | 50 | 10 | 3.50±0.53 | 3.40±0.52 | 6.59 |
| Sodium 2-(α-n-pentanonyl) benzoate | 50 | 10 | 3.40±0.70 | 3.00±0.67* | 17.58 |
| Potassium 2-(α-n-pentanonyl)benzoate | 50 | 11 | 3.27±0.79 | 2.91±0.70* | 20.05 |
| Calcium 2-(α-n-pentanonyl) benzoate | 50 | 11 | 3.18±0.60 | 2.73±0.65**,s | 25.00 |
| N,N'-dibenzylethylenediamine 2-(α-n-pentanonyl)benzoate | 50 | 11 | 3.45±0.69 | 2.91±0.70* | 20.05 |
| DL-3-n-butylphthalide | 50 | 11 | 3.36±0.81 | 3.09±0.54* | 15.11 |
| Potassium 2-(α-hydroxypentyl)benzoate | 50 | 10 | 3.50±0.71 | 3.30±0.48 | 9.34 |
| Calcium 2-(α-hydroxypentyl) benzoate | 50 | 11 | 3.45±0.52 | 3.36±0.50 | 7.69 |
| N,N'-dibenzylethylenediamine 2-(α-hydroxypentyl)benzoate | 50 | 11 | 3.45±0.52 | 3.09±0.54* | 15.11 |
| Note: ** P<0.01, * P<0.05 vs. model group.<br>s P<0.05, Calcium 2-(α-n-pentanonyl)benzoate vs. Calcium 2-(α-hydroxy pentyl)benzoate | | | | | |

Table 4. Effects of compounds (100 mg/kg) on the neural symptoms of MCAT rats

| Groups | Dosage (mg/kg) | n | Scores | | % inhibition at hour 24 |
|---|---|---|---|---|---|
| | | | 6 hours | 24 hours | |
| Model group | - | 11 | 3.64±0.50 | 3.73±0.47 | - |
| Pseudo-operation group | - | 11 | 0.00±0.00*** | 0.00±0.00*** | - |
| 3-n-butenylphthalide | 100 | 11 | 3.36±0.81 | 2.91±0.70** | 21.98 |

(continued)

| Groups | Dosage (mg/kg) | n | Scores | | % inhibition at hour 24 |
|---|---|---|---|---|---|
| | | | 6 hours | 24 hours | |
| Sodium 2-(α-n-pentanonyl) benzoate | 100 | 10 | 3.10±0.74 | 2.80±0.79** | 24.93 |
| Potassium 2-(α-n-pentanonyl) benzoate | 100 | 11 | 3.36±0.67 | 2.82±0.75** | 24.40 |
| Calcium 2-(α-n-pentanonyl) benzoate | 100 | 11 | 3.40±0.52 | 2.90±0.57** | 22.25 |
| N,N'-dibenzylethylenediamine 2-(α-n-pentanonyl)benzoate | 100 | 11 | 3.45±0.52 | 2.36±0.67 | 36.73 |
| DL-3-n-butylphthalide | 100 | 11 | 3.36±0.50 | 3.09±0.70* | 17.16 |
| Potassium 2-(α-hydroxypentyl)benzoate | 100 | 11 | 3.55±0.52 | 3.00±0.89* | 19.57 |
| Calcium 2-(α-hydroxypentyl) benzoate | 100 | 11 | 3.55±0.52 | 3.09±0.70* | 17.16 |
| N,N'-dibenzylethylenediamine 2-(α-hydroxypentyl)benzoate | 100 | 11 | 3.45±0.52 | 2.91±0.70** | 21.98 |
| Note: *** P<0.001, **P<0.01, * P<0.05 vs. model group. | | | | | |

Table 5. Effects of compounds (200 mg/kg) on the neural symptoms of MCAT rats

| Groups | Dosage (mg/kg) | n | Scores | | % inhibition at hour 24 |
|---|---|---|---|---|---|
| | | | 6 hours | 24 hours | |
| Model group | - | 11 | 3.55±0.52 | 3.73±0.47 | - |
| Pseudo-operation group | - | 11 | 0.00±0.00*** | 0.00±0.00*** | - |
| 3-n-butenylphthalide | 200 | 11 | 3.36±0.67 | 3.00±0.63** | 19.57 |
| Sodium 2-(α-n-pentanonyl) benzoate | 200 | 11 | 3.27±0.65 | 2.82±0.40*** | 24.40 |
| Potassium 2-(α-n-pentanonyl)benzoate | 200 | 10 | 3.20±0.63 | 2.10±0.32*** s | 43.70 |
| Calcium 2-(α-n-pentanonyl) benzoate | 200 | 11 | 3.27±0.79 | 2.45±0.82*** | 34.32 |
| N,N'-dibenzylethylenediamine 2-(α-n-pentanonyl)benzoate | 200 | 11 | 3.27±0.79 | 2.55±0.52*** | 31.64 |
| DL-3-n-butylphthalide | 200 | 11 | 3.36±0.67 | 3.00±0.89* | 19.57 |
| Potassium 2-(α-hydroxypentyl)benzoate | 200 | 11 | 3.45±0.52 | 2.73±0.79 ** | 26.81 |
| Calcium 2-(α-hydroxypentyl) benzoate | 200 | 11 | 3.45±0.52 | 2.91±0.54** | 21.98 |

(continued)

| Groups | Dosage (mg/kg) | n | Scores | | % inhibition at hour 24 |
|---|---|---|---|---|---|
| | | | 6 hours | 24 hours | |
| N,N'-dibenzylethylenediamine 2-(α-hydroxypentyl) benzoate | 200 | 11 | 3.36±0.67 | 2.82±0.87** | 24.40 |
| Note: *** P<0.001, ** P<0.01, * P<0.05 vs. model group. $^s$ P<0.05, Potassium 2-(α-n-pentanonyl)benzoate vs. potassium 2-(α-hydroxy pentyl)benzoate | | | | | |

The results showed that no abnormal behavioral was observed in pseudo-operation group, and hemiplegia like symptoms were observed in model rats at 6 hours and 24 hours after the operation, mainly exhibited as contralateral forelimbs adduction, shoulder intorsion, decreased tensile force of forelimbs muscle, and decreased should resistance. As compared with the model group, alleviation on neural symptoms at 24 hours after the operation was observed for all tested compounds in a dosage range of 50 to 200mg/kg body weight, but the extent of alleviation is different. In particularly, sodium, potassium, calcium and N,N'-dibenzylethylenediamine 2-(α-n-penta nonyl)benzoates; DL-3-n-butylphthalide; as well as N,N'-dibenzylethylenediamine 2-(α-hydroxypentyl)benzoate at the dosage of 50 mg/kg all exhibit significantly alleviation on neural symptoms at 24 hours after the operation (P<0.01 or P<0.05). All of the compounds at the dosages of 100 mg/kg and 200 mg/kg were shown to significantly alleviate the neural symptoms of rats at 24 hours after the operation (P<0.05, P<0.01 or P<0.001). The alleviation effect of calcium 2-(α-n-pentanonyl)benzoate at the dosage of 50 mg/kg on the neural symptoms of rats at 24 hours after the operation was significantly superior to that of calcium 2-(α-hydroxypentyl)benzoate (P<0.05). The alleviation effect of potassium 2-(α-n-pentanonyl)benzoate at the dosage of 200 mg/kg on the neural symptoms of rats at 24 hours after the operation was significantly superior to that of potassium 2-(α-hydroxypentyl)benzoate (P<0.05).

The results suggest that 2-(α-n-pentanonyl)benzoates has significant effect on reducing the tensile force of the limbs induced by ischemic cerebral injury. As compared with the known 2-(α-hydroxypentyl)benzoates, the compounds of the present invention has lower dosage to exhibit significant therapeutic effect. That is to say, the compounds of the present invention may exhibit significant effect at a lower dosage (50 mg/kg). Moreover, considering % inhibition at hour 24, the alleviation effects of potassium, calcium and N,N'-dibenzylethylenediamine 2-(α-n-pentanonyl)benzoates on the neural symptoms of MCAT rats were all higher than those of potassium, calcium and N,N'-dibenzylethylenediamine 2-(α-hydroxypentyl)benzoates at the same dosage.

B. Effects on cerebral infarct area of MCAT rats

After the final grading of the behavioral scores, the rats were decapitated and the brain was then obtained. The olfactory bulb, cerebellum and lower brain stem were removed, and the remain portion was cut into five coronal slices at a temperature of 4˚C. The slices were then immediately placed into TTC staining solution (5 ml of the solution containing 1.5 ml of 4% TTC and 0.1 ml of 1M $K_2HPO_4$). After lightproof incubation at 37˚C for 30 minutes, the slices were taken out and then placed into 10% formaldehyde solution for lightproof storage. After staining with TTC, the non-ischemic region showed rose color, while the infarct region showed white. The white tissue was carefully excavated and weighed, and the cerebral infarct area was expressed as percentage of cerebral infarct tissue weight to the total brain weight. The results in each group were compared by student t test, and were shown in tables 6, 7, and 8.

Table 6. Effects of compounds (50 mg/kg) on the cerebral infarct area of MCAT rats

| Groups | Dosage(mg/kg) | n | Cerebral infarct tissue weight/total brain weight (%) | Inhibition (%) |
|---|---|---|---|---|
| Model group | - | 11 | 7.41±2.17 | - |
| Pseudo-operation group | - | 11 | 0.00±0.00*** | - |
| 3-n-butenylphthalide | 50 | 10 | 5.90±1.50 | 20.38 |
| Sodium 2-(α-n-pentanonyl) benzoate | 50 | 10 | 5.26±2.26* | 29.01 |

(continued)

| Groups | Dosage(mg/kg) | n | Cerebral infarct tissue weight/total brain weight (%) | Inhibition (%) |
|---|---|---|---|---|
| Potassium 2-(α-n-pentanonyl) benzoate | 50 | 11 | 5.63±1.52* | 24.02 |
| Calcium 2-(α-n-pentanonyl) benzoate | 50 | 11 | 5.01±1.79* | 32.39 |
| N,N'-dibenzylethylenediamine 2-(α-n-pentanonyl)benzoate | 50 | 11 | 5.14±2.17* | 30.63 |
| DL-3-n-butylphthalide | 50 | 11 | 5.66±2.81 | 23.62 |
| Potassium 2-(α-hydroxypentyl) benzoate | 50 | 10 | 6.16±2.46 | 16.87 |
| Calcium 2-(α-hydroxypentyl) benzoate | 50 | 11 | 6.39±2.33 | 13.77 |
| N,N'-dibenzylethylenediamine 2-(α-hydroxypentyl)benzoate | 50 | 11 | 5.85±2.32 | 21.05 |
| Note: ** P<0.01, * P<0.05 vs. model group. | | | | |

Table 7. Effects of compounds (100 mg/kg) on the cerebral infarct area of MCAT rats

| Groups | Dosage(mg/kg) | n | Cerebral infarct tissue weight/total brain weight (%) | Inhibition (%) |
|---|---|---|---|---|
| Model group | - | 11 | 7.94±2.65 | - |
| Pseudo-operation group | - | 11 | 0.00±0.00*** | - |
| 3-n-butenylphthalide | 100 | 11 | 5.6.5±2.10* | 28.84 |
| Sodium 2-(α-n-pentanonyl) benzoate | 100 | 10 | 4.99±1.83** | 37.16 |
| Potassium 2-(α-n-Pentanonyl) benzoate | 100 | 11 | 5.09±2.04 * | 35.89 |
| Calcium 2-(α-n-pentanonyl) benzoate | 100 | 11 | 5.23±2.04* | 34.13 |
| N,N'-dibenzylethylenediamine 2-(α-n-pentanonyl)benzoate | 100 | 11 | 4.68±1.69* | 41.06 |
| DL-3-n-butylphthalide | 100 | 11 | 5.81±1.49* | 26.83 |
| Potassium 2-(α-hydroxypentyl) benzoate | 100 | 11 | 5.73±2.20* | 27.83 |
| Calcium 2-(α-hydroxypentyl) benzoate | 100 | 11 | 5.82±1.90* | 26.70 |
| N,N'-dibenzylethylenediamine 2-(α-hydroxypentyl)benzoate | 100 | 11 | 5.50±1.82* | 30.73 |
| Note: ** P<0.01, * P<0.05 vs. model group. | | | | |

Table 8. Effects of compounds (200 mg/kg) on the cerebral infarct area of MCAT rats

| Groups | Dosage(mg/kg) | n | Cerebral infarct tissue weight/total brain weight (%) | Inhibition (%) |
|---|---|---|---|---|
| Model group | - | 11 | 7.61±2.16 | - |
| Pseudo-operation group | - | 11 | 0.00±0.00*** | - |
| 3-n-butenylphthalide | 200 | 11 | 5.49±2.14* | 27.86 |
| Sodium 2-(α-n-pentanonyl) benzoate | 200 | 11 | 5.19±2.40* | 31.80 |
| Potassium 2-(α-n-pentanonyl) benzoate | 200 | 10 | 4.38±1.54*** | 42.44 |
| Calcium 2-(α-n-pentanonyl) benzoate | 200 | 11 | 4.65±2.13** | 38.90 |
| N,N'-dibenzylethylenediamine 2-(α-n-pentanonyl)benzoate | 200 | 11 | 4.60±1.26** | 39.55 |
| DL-3-n-butylphthalide | 200 | 11 | 5.52±1.78* | 27.46 |
| Potassium 2-(α-hydroxypentyl) benzoate | 200 | 11 | 5.37±2.00* | 29.43 |
| Calcium 2-(α-hydroxypentyl) benzoate | 200 | 11 | 5.41±1.77* | 28.91 |
| N,N'-dibenzylethylenediamine 2-(α-hydroxypentyl)benzoate | 200 | 11 | 5.09±2.14* | 33.11 |
| Note: *** P<0.001, ** P<0.01 , * P<0.05 vs. model group. | | | | |

The results showed that no infarction was observed in pseudo-operation group at 24 hours after the operation, while different extent of infarction was observed in the model group and in the test groups. The cerebral infarct of rats in the groups of sodium, potassium, calcium and N,N'-dibenzylethylenediamine 2-(α-n-pentanonyl)benzoates at the dosage of 50 mg/kg was significantly reduced as compared with the model group (P<0.05); The cerebral infarct of rats in the groups of each compound at the dosage of 100 mg/kg was smaller than that in the model group, and the differences from the model group have significance (P<0.01 or P<0.05); The cerebral infarct of rats in the groups of each compound at the dosage of 200 mg/kg was smaller than that in the model group, and the differences from the model group have significance (P<0.05, P<0.01 or P<0.001).

The results suggest that 2-(α-n-pentanonyl)benzoates has significant effect on reducing the cerebral infarct induced by ischemic cerebral injury. As compared with the known 2-(α-hydroxypentyl)benzoates, the compounds of the present invention has lower dosage to exhibit significant therapeutic effect. That is to say, the compounds of the present invention may exhibit significant effect at a lower dosage (50 mg/kg). Moreover, considering % inhibition, the alleviation effects of potassium, calcium and N,N'-dibenzylethylenediamine 2-(α-n-pentanonyl)benzoates on the cerebral infarct of MCAT rats were all higher than those of potassium, calcium and N,N'-dibenzylethylenediamine 2-(α-hydroxypentyl) benzoates at the same dosage.

Example 23: Effects on thrombosis of artery-vein bypass in rats

This example used the following:

1. Animals

Female and male SD rats, weighed 240-280g, were provided by Vital River, Beijing. The certificate of approval was SCXK 11-00-0008.

2. Drugs and reagents

Test drugs: 3-n-butenylphthalide, sodium, potassium, calcium and N,N'-dibenzylethylenediamine 2-(α-n-pentanonyl)benzoates were all provided by Teamacademy of Pharmaceutical Science, and were formulated into solutions of various concentrations with eatable salad oil.

Tian-Bao-Ning: a product of Zhejiang Conba Pharmaceutical Co., Ltd, Cat. No: 020108-4, the tested dosage of which was 24 mg/kg.

3. Instruments

Electronic analytical balance, model AEG-220, produced by SHIMADZU, Japan; Blast drying oven, model Df-206, produced by Beijing Xi-Cheng Medical Instrument Plant.

4. Testing methods and results

Testing methods are described in Chen-Qi, "Methodology for Pharmacological Research of Chinese Medicine", People's Medical Publishing House, September 1993, 1st ed., page 510.

(1) Grouping and administration

The rats were randomly divided into the following groups, which were thrombus model group, groups of 3-n-butenylphthalide at the dosages of 200 mg/kg, 100 mg/kg and 50 mg/kg; groups of sodium 2-(α-n-pentanonyl)benzoate at the dosages of 200 mg/kg, 100 mg/kg and 50 mg/kg; groups of potassium 2-(α-n-pentanonyl)benzoate at the dosages of 200 mg/kg, 100 mg/kg and 50 mg/kg; groups of calcium 2-(α-n-pentanonyl)benzoate at the dosages of 200 mg/kg, 100 mg/kg and 50 mg/kg; groups of N,N'-dibenzylethylenediamine 2-(α-n-pentanonyl)benzoate at the dosages of 200 mg/kg, 100 mg/kg and 50 mg/kg; group of Tian-Bao-Ning at the dosage of 24 mg/kg. Each administration group was administered by gavage at a dosage of 0.2 ml per 100 g of body weight, one time per day for 3 days. Equivalent amount of the vehicle was administered to the thrombus model group. The operation was conducted immediately after the final administration.

2. The Method for Establishing the Model

Rats were anesthetized by intraperitoneally injecting 10% chloral hydrate (0.35 ml per 100 g), and fixed in lateral decubitus position. The operation was conducted to separate the right common carotid artery and the left external jugular vein. A pre-weighed suture # 0 of 8 cm long was placed into the middle part of a polyethylene tube of 10 cm long. Then the tube was filled with normal saline, and connected at both ends to the cannula of 3 cm long filled with normal saline. One end of the tube was inserted into the left external jugular vein, and another end was inserted into the right common carotid artery. The blood flowed from the right common carotid artery to the polyethylene tube, and then flow back to the left external jugular vein, so as to construct a bypass circulation. The blood flow was occluded after 10 minutes, the thrombus was taken out and then weighed immediately to obtain a total wet weight. The wet-weight of the thrombus was obtained by subtracting the weight of the suture and the paper from this total wet weight. The thrombus was placed into an oven of 60 ˚C to dry for 24 hours. The thrombus was taken out and weighed to obtain a total dry weight. The dry weight of the thrombus was obtained by subtracting the weight of the suture and the paper form this total dry weight. The results in each group were compared by student's t test. The % inhibition was calculated by using the following equation:

$$\%\ \text{Inhibition} = \frac{\text{thrombus weight of model group} - \text{thrombus weight of test group}}{\text{thrombus weight of model group}}$$

3. Testing results

The results were shown in table 9 and 10.

Table 9. Inhibitory effects of 3-n-butenylphthalide and sodium 2-(α-n-pentanonyl)benzoate on thrombus of artery-vein bypass in rats **($\bar{X}\pm SD$)**

| Groups | Dosage (mg/kg) | n | Wet-weight of thrombus (mg) | Inhibition (%) | Dry-weight of thrombus (mg) | Inhibition (%) |
|---|---|---|---|---|---|---|
| Thrombus model group | - | 12 | 211.3±84.6 | - | 38.0±15.2 | - |
| 3-n-butenylphthalide | 200 | 10 | 141.6±41.2* | 32.99 | 24.9±12.3* | 34.47 |
| | 100 | 10 | 137.5±47.4* | 34.93 | 22.9±11.9* | 39.74 |
| | 50 | 10 | 169.9±28.8 | 19.59 | 28.6±9.6 | 24.74 |
| sodium 2-(α-n-pentanonyl) benzoate | 200 | 10 | 131.1±48.0* | 37.96 | 22.4±10.1* | 41.05 |
| | 100 | 10 | 115.4±42.3** | 45.39 | 19.7±7.8** | 48.16 |
| | 50 | 10 | 138.8±41.1* | 34.31 | 26.2±9.7 | 31.05 |
| Tian-bao-Ning | 24 | 11 | 134.3±43.6* | 36.44 | 25.4±10.3* | 33.16 |
| Note: ** P<0.01 , * P<0.05 vs. thrombus model group. | | | | | | |

Table 10. Inhibitory effects of potassium, calcium and N,N'-dibenzylethylenediamine 2-(α-n-pentanonyl)benzoate on thrombus of artery-vein bypass in rats **($\bar{X}\pm SD$)**

| Groups | Dosage (mg/kg) | n | Wet-weight of thrombus (mg) | Inhibition (%) | Dry-weight of thrombus (mg) | Inhibition (%) |
|---|---|---|---|---|---|---|
| Thrombus model group | - | 10 | 259.2±67.1 | - | 51.3±12.9 | - |
| Potassium 2-(α-n-pentanonyl)benzoate | 200 | 10 | 157.0±51.4** | 39.43 | 28.3±16.3** | 44.8 |
| | 100 | 10 | 192.6±41.2* | 25.69 | 35.6±14.0* | 30.60 |
| | 50 | 10 | 208.8±45.3 | 19.44 | 38.3±15.4 | 25.34 |
| Calcium 2-(α-n-pentanonyl)benzoate | 200 | 11 | 163.2±64.4** | 37.04 | 30.4±13.9** | 40.74 |
| | 100 | 10 | 189.8±52.3* | 26.77 | 34.2±13.1* | 33.33 |
| | 50 | 10 | 199.8±30.4* | 22.92 | 40.2±18.0 | 21.64 |
| N,N'-dibenzylethylenediamine 2-(α-n-pentanonyl) benzoate | 200 | 10 | 191.7±34.8* | 26.04 | 32.3±12.5** | 37.04 |
| | 100 | 10 | 157.9±67.9** | 39.08 | 29.9±13.6** | 41.72 |
| | 50 | 10 | 207.1±45.3 | 20.10 | 39.0±10.8* | 23.98 |
| Note: ** P<0.01, * P<0.05 vs. thrombus model group. | | | | | | |

The results showed that each compound at a dosage ranging from 50 to 100 mg/kg had inhibitory effect on thrombosis, and decrease the wet-weight and dry-weight of thrombus of artery-vein bypass at different extent. The maximum % inhibition of 3-n-butenylphthalide at the dosage of 100 mg/kg on the wet-weight and dry-weight of thrombus was up to 34.93% and 39.74%, respectively; The maximum % inhibition of sodium 2-(α-n-pentanonyl)benzoate at the dosage of 100 mg/kg on the wet-weight and dry-weight of thrombus was up to 45.39% and 48.16%, respectively; The maximum % inhibition of potassium 2-(α-n-pentanonyl)benzoate at the dosage of 200 mg/kg on the wet-weight and dry-weight of thrombus was up to 39.43% and 44.83%, respectively; The maximum % inhibition of calcium 2-(α-n-pentanonyl) benzoate at the dosage of 200 mg/kg on the wet-weight and dry-weight of thrombus was up to 37.04% and 40.74%, respectively; The maximum % inhibition of N,N'-dibenzylethylenediamine 2-(α-n-pentanonyl)benzoate at the dosage of 100 mg/kg on the wet-weight and dry-weight of thrombus was up to 39.08% and 41.72%, respectively.

The results suggest that the significant alleviating effects of 3-n-butenylphthalide and the above-mentioned 2-(α-n-pentanonyl)benzoates on the neural symptoms and cerebral infarct area of MCAT rats may be related to their inhibitory effects on thrombosis.

**Claims**

1. 2-(α-n-pentanonyl)benzoates having the following formula

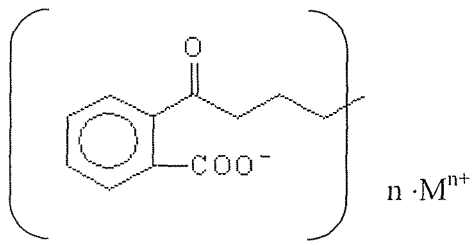

wherein n is 1 or 2; M is a monovalent metal ion, a bivalent metal ion or an organic base group.

2. 2-(α-n-pentanonyl)benzoates as claimed in claim 1, wherein M is a monovalent metal ion selecting from the group consisting of $Li^+$, $Na^+$ and $K^+$, or a bivalent metal ion selecting from the group consisting of $Mg^{2+}$, $Ca^{2+}$ and $Zn^{2+}$, or an organic base group selecting from the group consisting of benzyl amine, t-butyl amine, methyl benzyl amine and N,N'-dibenzylethylenediamine.

3. 2-(α-n-pentanonyl)benzoates as claimed in claim 1, wherein M is selected from the group consisting of $Na^+$, $K^+$, $Ca^{2+}$, N,N'-dibenzylethylenediamine.

4. A method for preparing 2-(α-n-pentanonyl)benzoates as claimed in claim 1, wherein M is an organic base group, said method comprises:

hydrolyzing 3-n-butenylphthalide under an alkaline condition; acidifying the hydrolyzed product to obtain 2-(α-n-pentanonyl)benzoic acid; dissolving the 2-(α-n-pentanonyl)benzoic acid in a solvent with low polarity and then reacting with an organic base; salting out, filtering, washing and drying to obtain 2-(α-n-pentanonyl)benzoates, wherein the solvent with low polarity comprises benzenes, ethers, dichloromethane, and ethyl acetate.

5. The method for preparing 2-(α-n-pentanonyl)benzoates as claimed in claim 1, wherein M is a monovalent metal ion, said method comprises:

hydrolyzing 3-n-butenylphthalide under an alkaline condition; acidifying the hydrolyzed product to obtain 2-(α-n-pentanonyl)benzoic acid; reacting the 2-(α-n-pentanonyl)benzoic acid with a metal ionic base dissolved in a solvent with high polarity to form a salt, and then adding a solvent with low polarity under stirring, stirring for several hours, salting out, filtering, washing with solvent, drying to obtain 2-(α-n-pentanonyl)benzoates, wherein the solvent with high polarity comprises C1-C4 lower alcohols, and wherein the solvent with low polarity comprises benzenes, ethers, dichloromethane, and ethyl acetate.

6. The method as claimed in claim 4 or 5, wherein the solvent with low polarity is ethyl ether, and the solvent with high polarity is methanol.

7. The method for preparing 2-(α-n-pentanonyl)benzoates as claimed in claim 1, wherein M is a bivalent metal ion, said method comprises mixing a solution of 2-(α-n-pentanonyl)benzoates with a solution of bivalent metal ion salt, performing trans-salification to obtain 2-(α-n-pentanonyl)benzoates of bivalent metal ion.

8. Use of 2-(α-n-pentanonyl)benzoates as claimed in claim 1 in preparing the medicament for treating or preventing cardio-cerebral ischemic diseases, alleviating the disturbance of cardio-cerebral circulation, and inhibiting thrombosis.

9. A pharmaceutical composition for treating and preventing cardio-cerebral ischemic diseases, alleviating the disturbance of cardio-cerebral circulation and inhibiting thrombosis, which comprises a therapeutically effective amount of 2-(α-n-pentanonyl)benzoates as claimed in claim 1, and one or more pharmaceutically acceptable carriers.

**10.** The pharmaceutical composition as claimed in claim 9, which is formulated into tablets, capsules, granules, intravenous injections, or lyophilized intravenous injections.

# INTERNATIONAL SEARCH REPORT

International application No. PCT/CN2004/000602

A. CLASSIFICATION OF SUBJECT MATTER

IPC[7] C07C65/32 C07C51/00 A61K31/19 A61P9/10

According to International Patent Classification (IPC) or to both national classification and IPC

B. FIELDS SEARCHED

Minimum documentation searched (classification system followed by classification symbols)

C07C A61K A61P

Documentation searched other than minimum documentation to the extent that such documents are included in the fields searched

Electronic data base consulted during the international search (name of data base and, where practicable, search terms used)

WPI, EPODOC, PAJ, CNPAT, CA ,STN

C. DOCUMENTS CONSIDERED TO BE RELEVANT

| Category* | Citation of document, with indication, where appropriate, of the relevant passages | Relevant to claim No. |
|---|---|---|
| A | CN1382682 A 04.Dec.2002 (2002-12-04)<br>See the whole documents | 1-10 |
| A | JP1199958 A 11.Aug.1989(1989-08-11)<br>See the whole documents | 1-10 |
| A | JP55098169 A 25.Jul.1980(1980-07-25)<br>See the whole documents | 1-10 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 19.11.2004 | 27 · JAN 2004 (27 · 01 · 2004) |
| Name and mailing address of the ISA/CN<br>6 Xitucheng Rd., Jimen Bridge, Haidian District, 100088 Beijing, China<br>Facsimile No. 86-10-62019451 | Authorized officer<br>Luo Ling<br>Telephone No. 86-10-62085605 |

Form PCT/ISA /210 (second sheet) (January 2004)

**INTERNATIONAL SEARCH REPORT**
Information on patent family members

International application No.
PCT/CN2004/000602

| Patent document cited In search report | Publication date | Patent family member(s) | Publication date |
|---|---|---|---|
| CN1382682 A | 04.12.2002 | NONE | |
| JP1199958 A | 11.08.1989 | NONE | |
| JP55098169 A | 25.07.1980 | NONE | |

Form PCT/ISA /210 (patent family annex) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN ZL9A117148
- CN 01109795


### Non-patent literature cited in the description


- *Biochimica et, Biophysica Acta,* 1987, vol. 924, 375-382
- *Journal of Lanzhou University,* 1990, vol. 26 (1), 118-119
- **TAMURA A ; GRAHAM DI ; MCCULLUOCH J et al.** Focal Cerebral ischemia in the rat. 1. Description of technique and early neuropathological consequences following middle cerebral artery occlusion. *J Cereb Blood Flow Metab,* 1981, vol. 1, 53
- **CHEN-QI.** Methodology for Pharmacological Research of Chinese Medicine. People's Medical Publishing House, September 1993, 510